(19) Europäisches Patentamt
European Patent Office
Office européen des brevets

(11) **EP 1 790 338 A1**

(12) **EUROPEAN PATENT APPLICATION**
published in accordance with Art. 158(3) EPC

(43) Date of publication:
**30.05.2007 Bulletin 2007/22**

(21) Application number: **05783155.4**

(22) Date of filing: **13.09.2005**

(51) Int Cl.:
*A61K 31/192* (2006.01)      *A61K 31/421* (2006.01)
*A61K 31/603* (2006.01)      *A61P 27/02* (2006.01)
*A61P 43/00* (2006.01)       *C07D 263/32* (2006.01)

(86) International application number:
**PCT/JP2005/016788**

(87) International publication number:
**WO 2006/030753 (23.03.2006 Gazette 2006/12)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.09.2004 JP 2004265197**

(71) Applicant: **SANTEN PHARMACEUTICAL CO., LTD.**
**Higashiyodogawa-ku,**
**Osaka-shi,**
**Osaka 533-8651 (JP)**

(72) Inventors:
• **NAKAMURA, M.,**
**SANTEN PHARMACEUTICAL CO., LTD.**
**Ikoma-shi,**
**Nara 6300101 (JP)**

• **HIRAI, S.,**
**SANTEN PHARMACEUTICAL CO., LTD.**
**Ikoma-shi,**
**Nara 6300101 (JP)**
• **SHIBAGAKI, K.,**
**SANTEN PHARMACEUTICAL CO., LTD.**
**Ikoma-shi,**
**Nara 6300101 (JP)**

(74) Representative: **Müller-Boré & Partner**
**Patentanwälte**
**Grafinger Straße 2**
**D-81671 München (DE)**

(54) **THERAPEUTIC AGENT FOR KERATOCONJUNCTIVA DISORDER**

(57)    An object of the present invention is to research a new medicinal use of E-4-[4-(5-methyl-2-phenyl-4-oxazolylmethoxy) benzyloxyimino]-4-phenylbutyric acid, Z-2-[4-(5-methyl-2-phenyl-4-oxazolylmethoxy) benzyloxyimino]-2-(4-phenoxyphenyl)acetic acid, 2-[2-propyl-3-[3-[2-ethyl-4-(4-fluorophenyl)-5-hydroxyphenoxy]propoxy]phenoxy] benzoic acid, 2(S)-methoxy-3-[4-[3-(4-phenoxyphenoxy)propoxy]phenyl] propionic acid, or a salt thereof. Any of the above-mentioned carboxylic acid compounds and a salt thereof exhibit an excellent improving effect on corneal disorder models and are useful as a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

EP 1 790 338 A1

**Description**

Technical Field

The present invention relates to a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis, comprising as an active ingredient, at least one of (1)E-4-[4-(5-methyl-2-phenyl-4-oxazolylmethoxy) benzyloxyimino]-4-phenylbutyric acid, (2)Z-2-[4-(5-methyl-2-phenyl-4-oxazolylmethoxy) benzyloxyimino]-2-(4-phenoxyphenyl)acetic acid, (3)2-[2-propyl-3-[3-[2-ethyl-4-(4-fluorophenyl)-5-hydroxyphenoxy]propoxy]phenoxy] benzoic acid, (4)2(S)-methoxy-3-[4-[3-(4-phenoxyphenoxy)propoxy]phenyl] propionic acid, or a salt thereof.

Background Art

Cornea is a transparent avascular tissue having a diameter of about 1 cm and a thickness of about 1 mm, while conjunctiva is a mucosal membrane covering the eyeball surface posterior to the corneal margin, and the back face of the eyelid. The cornea and the conjunctiva are known to significantly affect the visual function. Keratoconjunctival disorders caused due to a variety of diseases such as corneal ulcer, keratitis, conjunctivitis, dry eyes and the like may adversely affect normal architecture of epithelium, and furthermore, may impair structures and functions of the stroma and endothelium, when the repair of these disorders is retarded, alternatively when these disorders are prolonged without making repair on some grounds. That is because the cornea and the conjunctiva are connected tissues. In these years, with the development of cell biology, factors participating in cell proliferation, migration, adhesion, extension, differentiation and the like had been elucidated, and it was reported that these factors play important roles in repair of corneal disorders (Japanese Review of Clinical Ophthalmology, 46, 738-743 (1992), Ophthalmic Surgery, 5, 719-727 (1992)).

On the other hand, Japanese Patent No. 3074532 discloses that an oxyiminoalkanoic acid derivative such as E-4-[4-(5-methyl-2-phenyl-4-oxazolylmethoxy) benzyloxyimino]-4-phenylbutyric acid or Z-2-[4-(5-methyl-2-phenyl-4-oxazolylmethoxy)benzyloxyimino]-2-(4-phenoxyphenyl) acetic acid is effective as a therapeutic agent or a preventive for diabetes, hyperlipidemia or the like. Japanese Patent No. 3417582 and International Publication WO 2002/100813 suggest that a leukotriene-antagonistic substituted phenylphenol such as 2-[2-propyl-3-[3-[2-ethyl-4-(4-fluorophenyl)-5-hydroxyphenoxy]propoxy]phenoxy] benzoic acid or 2(S)-methoxy-3-[4-[3-(4-phenoxyphenoxy)propoxy]phenyl] propionic acid is effective as a therapeutic agent for inflammatory diseases and allergic diseases.

However, there has been no report in which a pharmacological effect of these carboxylic acid compounds on an eye disease such as a keratoconjunctival disorder is studied.

Disclosure of the Invention

Problem to be Solved

It is an interesting subject to research a new medicinal use of E-4-[4-(5-methyl-2-phenyl-4-oxazolylmethoxy) benzyloxyimino]-4-phenylbutyric acid, Z-2-[4-(5-methyl-2-phenyl-4-oxazolylmethoxy) benzyloxyimino]-2-(4-phenoxyphenyl)acetic acid, 2-[2-propyl-3-[3-[2-ethyl-4-(4-fluorophenyl)-5-hydroxyphenoxy]propoxy]phenoxy] benzoic acid, 2(S)-methoxy-3-[4-[3-(4-phenoxyphenoxy)propoxy]phenyl] propionic acid, or a salt thereof.

Means of Solving Problems

The present inventors have made intensive studies in order to research a new medicinal use of the above-mentioned carboxylic acid compounds, and as a result, they found that all of these compounds exhibit an excellent improving effect on a corneal damage in a test for therapeutic effect using corneal disorder models and thus the present invention has been accomplished.

That is, the present invention is directed to a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis, comprising as an active ingredient, at least one of (1)E-4-[4-(5-methyl-2-phenyl-4-oxazolylmethoxy) benzyloxyimino]-4-phenylbutyric acid, (2)Z-2-[4-(5-methyl-2- phenyl-4-oxazolylmethoxy) benzyloxyimino]-2-(4-phenoxyphenyl)acetic acid, (3) 2-[2-propyl-3-[3-[2-ethyl-4-(4-fluorophenyl)-5-hydroxyphenoxy]propoxy]phenoxy] benzoic acid, (4)2(S)-methoxy-3-[4-[3-(4-phenoxyphenoxy)propoxy]phenyl] propionic acid, or a salt thereof (hereinafter, these are referred to as "the present compounds").

The salt of the above-mentioned carboxylic acid compounds (1) to (4) is not particularly limited as long as it is a pharmaceutically acceptable salt. Examples thereof include sodium salts, potassium salts, lithium salts, calcium salts, magnesium salts, salts with an inorganic acid such as hydrochloric acid, nitric acid or sulfuric acid, salts with an organic acid such as acetic acid, fumaric acid, maleic acid, succinic acid or tartaric acid, and the like. Quaternary ammonium salts are also included in the salt according to the present invention. Preferred salts are sodium salts and potassium salts. The present compounds may be in a form of a hydrate or a solvate. A geometric isomer, optical isomer, racemate, tautomer or polymorphism of any of the present compounds may also be included in the scope of the present invention.

The keratoconjunctival disorder referred to herein means the state of damaged cornea and/or conjunctiva due to various factors, and examples thereof include dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis, filamentary keratitis and the like.

The therapeutic agent for a keratoconjunctival disorder of the present invention may be administered either orally or parenterally.

Examples of the dosage form include eyedrops, ophthalmic ointments, injections, tablets, capsules, granules, powders and the like. In particular, eyedrops are preferred. These can be prepared using any of generally used techniques. For example, the eyedrops can be prepared using a tonisity agent such as sodium chloride or concentrated glycerin, a buffer such as sodium phosphate or sodium acetate, a surfactant such as polyoxyethylene sorbitan monooleate, polyoxyl 40 stearate or polyoxyethylene hydrogenated castor oil, a stabilizer such as sodium citrate or sodium edetate, a preservative such as benzalkonium chloride or paraben as needed. The pH of the eyedrops is permitted as long as it falls within the range that is acceptable as an ophthalmic preparation, but is preferably in the range of from 4 to 8.

The ophthalmic ointment can be prepared with a generally used base such as white soft paraffin or liquid paraffin. Also, oral preparations such as tablets, capsules, granules and powders can be prepared by adding an extender such as lactose, crystalline cellulose, starch or vegetable oil, a lubricant such as magnesium stearate or talc, a binder such as hydroxypropyl cellulose or polyvinyl pyrrolidone, a disintegrant such as carboxymethyl cellulose calcium or low-substituted hydroxypropylmethyl cellulose, a coating agent such as hydroxypropylmethyl cellulose, macrogol or a silicone resin, a film forming agent such as gelatin film, and the like, as needed.

The present invention also relates to a method for treating a keratoconjunctival disorder comprising administering to a patient a therapeutically effective amount of at least one of the above-mentioned carboxylic acid compounds (1) to (4) or a salt thereof.

The dose can be properly selected depending on the symptoms, age, dosage form and the like. In the case of an eyedrop, it may be instilled once to several times a day at a concentration of from 0.0001 to 5% (w/v), preferably from 0.001 to 3% (w/v). In the case of an oral preparation, it may be administered once or divided into several times at a dose of generally from 0.1 to 5000 mg per day, preferably from 1 to 1000 mg per day.

Advantage of the Invention

As will be described below, when a test for a therapeutic effect on a corneal damage was carried out, any of the present compounds were found to exhibit an excellent improving effect on corneal disorder models. Therefore they are useful as a therapeutic agent for a keratoconjunctival disorder such as dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

Best Mode for Carrying Out the Invention

Hereinafter, results of a pharmacological test and preparation examples using the present compounds will be shown, however, these examples are for understanding the present invention well, and are not meant to limit the scope of the invention.

[Pharmacological test]

Test for therapeutic effect on corneal damage

Using male SD rats, in accordance with the method of Fujihara et al. (Invest. Ophthalmol. Vis. Sci. 42 (1): 96-100 (2001)), corneal disorder models were produced. After the production of the corneal disorder models, the improvement ratio of a corneal damage was evaluated according to the method of Murakami et al. (Journal of the eye 21 (1) : 87-90 (2004)).

(Test method)

Male SD rats were systemically anesthetized by an administration of Nembutal. Subsequently the exorbital lacrimal gland of each rat was removed and a corneal damage was induced over a period of 2 months.

Then, E-4-[4-(5-methyl-2-phenyl-4-oxazolylmethoxy) benzyloxyimino]-4-phenylbutyric acid (Compound A), Z-2-[4-(5-methyl-2-phenyl-4-oxazolylmethoxy) benzyloxyimino]-2-(4-phenoxyphenyl) acetic acid (Compound B), 2-[2-propyl-3-[3-[2-ethyl-4-(4-fluorophenyl)-5-hydroxyphenoxy]propoxy]phenoxy]sodium benzoate (Compound C) or 2(S)-methoxy-3-[4-[3-(4-phenoxyphenoxy)propoxy]phenyl] propionic acid (Compound D) was administered to the rats as follows.

Compound A administration group:

A physiological saline solution of Compound A (0.006%) was instilled into both eyes 6 times a day for 7 days (one group consisting of 4 animals, 8 eyes).

Compound B administration group:

A physiological saline solution of Compound B (0.006%) was instilled into both eyes 6 times a day for 7 days (one group consisting of 4 animals, 8 eyes).

Compound C administration group:

A physiological saline solution of Compound C (0.006%) was instilled into both eyes 6 times a day for 7 days (one group consisting of 4 animals, 8 eyes).

Compound D administration group:

A phosphate-buffered saline (PBS) solution of Compound D (0.002%) was instilled into both eyes 6 times a day for 7 days (one group consisting of 4 animals, 8 eyes).

In a control group, physiological saline or PBS was instilled into both eyes 6 times a day for 7 days (one group consisting of 4 animals, 8 eyes).

Seven days after the start of instillation, the damaged parts of the cornea were stained with fluorescein. For each of the upper, middle and lower parts of the cornea, the degree of fluorescein staining was evaluated by scoring according to the criteria shown below and the mean value of the total scores for each of the above-mentioned parts was calculated.

(Evaluation criteria)

0: No punctate staining
1: Scattered staining (punctate staining being separated)
2: Moderate staining (a part of punctuate staining being adjacent)
3: Heavy staining (punctate staining being barely separated)

(Results)

By taking the mean value of the total scores for the control group (physiological saline or PBS) as a standard (improvement ratio: 0%), the calculation was carried out. Each of the improvement ratios for the Compound A administration group, the Compound B administration group and the Compound C administration group is shown in Table 1 and the improvement ratio for the Compound D administration group is shown in Table 2. The mean value of the scores is a mean of those of 8 cases, respectively. The improvement ratio was calculated according to the following equation.

```
Improvement  ratio  (%)  =  {(control)  -  (the  present

compound)} / damage degree × 100
```

```
Damage degree = { (control) - (normal eye) }
```

Table 1

| Group | Mean value of total scores | Improvement ratio (%) |
|---|---|---|
| Normal eye | 3.0 | |
| Control group | 6.1 | 0 |
| Compound A administration group | 5.1 | 32.3 |
| Compound B administration group | 5.1 | 32.3 |
| Compound C administration group | 4.9 | 38.7 |

* control group : physiological saline administration group

Table 2

| Group | Mean value of total scores | Improvement ratio (%) |
|---|---|---|
| Normal eye | 3.1 | |
| Control group | 5.6 | 0 |
| Compound D administration group | 5.3 | 12.0 |

* control group : PBS administration group

(Discussion)

As apparent from the results of the above pharmacological test using rats, all of the carboxylic acid compounds A to D significantly improve a corneal damage.

[Preparation examples]

Hereinafter, representative preparation examples using Compounds A to D will be shown.

Preparation example 1

In 100 ml,

| | |
|---|---|
| Compound A | 10 mg |
| Sodium Chloride | 900 mg |
| Sterile purified water | q.s. |

By altering the amount of Compound A to be added, an eyedrop at a concentration of 0.001% (w/v), 0.03% (w/v), 0.1% (w/v), 0.3% (w/v), 1.0% (w/v) or 3.0% (w/v) can be prepared.

Preparation example 2

In 100 ml,

| | |
|---|---|
| Compound B | 100 mg |
| Sodium Chloride | 800 mg |
| Disodium hydrogen phosphate | 100 mg |
| Sodium dihydrogen phosphate | q.s. |
| Sterile purified water | q.s. |

By altering the amount of Compound B to be added, an eyedrop at a concentration of 0.003% (w/v), 0.01% (w/v), 0.03% (w/v), 0.05% (w/v), 0.3% (w/v), 1% (w/v) or 3% (w/v) can be prepared.

Preparation example 3

In 100 ml,

| | |
|---|---|
| Compound C | 50 mg |
| Sodium Chloride | 800 mg |
| Disodium hydrogen phosphate | 100 mg |
| Sodium dihydrogen phosphate | q.s. |
| Sterile purified water | q.s. |

By altering the amount of Compound C to be added, an eyedrop at a concentration of 0.002% (w/v), 0.01% (w/v), 0.25% (w/v), 1.25% (w/v) or 3% (w/v) can be prepared.

Preparation example 4

In 100 ml,

| | |
|---|---|
| Compound D | 20 mg |
| Sodium Chloride | 800 mg |
| Disodium hydrogen phosphate | 100 mg |
| Sodium dihydrogen phosphate | q.s. |
| Sterile purified water | q.s. |

By altering the amount of Compound D to be added, an eyedrop at a concentration of 0.001% (w/v), 0.01% (w/v), 0.25% (w/v), 1.25% (w/v) or 3% (w/v) can be prepared.

Preparation example 5

In 100 g,

| | |
|---|---|
| Compound C | 0.3 g |
| Liquid paraffin | 10.0 g |
| White soft paraffin | q.s. |

By altering the amount of Compound C to be added, an ophthalmic ointment at a concentration of 1% (w/w) or 3% (w/w) can be prepared.

**Claims**

1. A therapeutic agent for a keratoconjunctival disorder comprising at least one of the following carboxylic acid com-

6

pounds (1) to (4) or a salt thereof as an active ingredient. (1)E-4-[4-(5-methyl-2-phenyl-4-oxazolylmethoxy) benzyloxyimino]-4-phenylbutyric acid; (2)Z-2-[4-(5-methyl-2-phenyl-4-oxazolylmethoxy) benzyloxyimino]-2-(4-phenoxyphenyl)acetic acid; (3)2-[2-propyl-3-[3-[2-ethyl-4-(4-fluorophenyl)-5-hydroxyphenoxy]propoxy]phenoxy] benzoic acid; (4)2(S)-methoxy-3-[4-[3-(4-phenoxyphenoxy)propoxy]phenyl] propionic acid

2. The therapeutic agent according to Claim 1, wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

3. The therapeutic agent according to Claim 1, which is in a dosage form of an eyedrop or an ophthalmic ointment.

4. A method for treating a keratoconjunctival disorder comprising administering to a patient a therapeutically effective amount of at least one of the following carboxylic acid compounds (1) to (4) or a salt thereof. (1)E-4-[4-(5-methyl-2-phenyl-4-oxazolylmethoxy) benzyloxyimino]-4-phenylbutyric acid; (2)Z-2-[4-(5-methyl-2-phenyl-4-oxazolylmethoxy) benzyloxyimino]-2-(4-phenoxyphenyl)acetic acid; (3)2-[2-propyl-3-[3-[2-ethyl-4-(4-fluorophenyl)-5-hydroxyphenoxy]propoxy]phenoxy] benzoic acid; (4)2(S)-methoxy-3-[4-[3-(4-phenoxyphenoxy)propoxy]phenyl] propionic acid

5. The therapeutic method according to Claim 4, wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

6. The therapeutic method according to Claim 4, wherein the dosage form is an eyedrop or an ophthalmic ointment.

7. A use of at least one of the following carboxylic acid compounds (1) to (4) or a salt thereof for manufacturing a therapeutic agent for a keratoconjunctival disorder. (1)E-4-[4-(5-methyl-2-phenyl-4-oxazolylmethoxy) benzyloxyimino]-4-phenylbutyric acid; (2)Z-2-[4-(5-methyl-2-phenyl-4-oxazolylmethoxy) benzyloxyimino]-2-(4-phenoxyphenyl) acetic acid; (3)2-[2-propyl-3-[3-[2-ethyl-4-(4-fluorophenyl)-5-hydroxyphenoxy]propoxy]phenoxy] benzoic acid; (4)2 (S)-methoxy-3-[4-[3-(4-phenoxyphenoxy)propoxy]phenyl] propionic acid

8. The use according to Claim 7, wherein the keratoconjunctival disorder is dry eyes, corneal ulcer, keratitis, conjunctivitis, superficial punctate keratopathy, corneal epithelial defects, conjunctival epithelial defects, keratoconjunctivitis sicca, superior limbic keratoconjunctivitis or filamentary keratitis.

9. The use according to Claim 7, wherein the dosage form is an eyedrop or an ophthalmic ointment.

## INTERNATIONAL SEARCH REPORT

| International application No. |
|---|
| PCT/JP2005/016788 |

A. CLASSIFICATION OF SUBJECT MATTER
*A61K31/192* (2006.01), *A61K31/421* (2006.01), *A61K31/603* (2006.01), *A61P27/02* (2006.01), *A61P43/00* (2006.01), *C07D263/32* (2006.01)

According to International Patent Classification (IPC) or to both national classification and IPC

B. FIELDS SEARCHED

Minimum documentation searched (classification system followed by classification symbols)
*A61K31/192* (2006.01), *A61K31/421* (2006.01), *A61K31/603* (2006.01), *A61P27/02* (2006.01), *A61P43/00* (2006.01), *C07D263/32* (2006.01)

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
REGISTRY(STN), CAplus(STN), BIOSIS(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| Y | JP 3074532 B2 (Takeda Chemical Industries, Ltd.), 09 June, 2000 (09.06.00), Claims 1, 2, 9; Par. Nos. [0002], [0045], [0068]; examples 1, 7 & WO 99/58510 A1 & JP 2000-198772 A & JP 2000-3034266 A & EP 1077957 A1 & US 6251926 B1 & US 6495581 B1 & US 2003/186985 A1 & EP 1428531 A1 & EP 1077957 B1 | 1-3,7-9 |
| Y | SAKAMOTO, J., et al., A novel oxyiminoalkanoic acid derivative, TAK-559, activates human peroxisome proliferators-activated receptor subtypes, Eur.J.Pharmacol., July, 2004, Vol.495, pages 17 to 26, abstract, Figs. 1, 2 | 1-3,7-9 |

| ☒ Further documents are listed in the continuation of Box C. | ☐ See patent family annex. |
|---|---|

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 November, 2005 (01.11.05) | 20 December, 2005 (20.12.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
| --- |
| PCT/JP2005/016788 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | WO 2000/000194 A1  (PHOTOGENESIS INC, US),<br>06 January, 2000 (06.01.00),<br>& US 6316465 B1<br>Field of the invention, page 1, lines 30 to 33;<br>page 6, lines 3 to 7; page 7, line 10 to<br>page 8, line 3; page 12, lines 9 to 12;<br>table 1 | 1-3,7-9 |
| A | JP 3417582 B2  (LILLY & CO ELI),<br>11 April, 2003 (11.04.03),<br>& EP 544488 A2         & EP 544488 A3<br>& US 5462954 A          & EP 544488 B1<br>& JP 05-286852 A | 1-3,7-9 |
| A | WO 2002/100813 A2  (LILLY & CO ELI),<br>19 December, 2002 (19.12.02),<br>& EP 1392637 A2          & US 2005/020684 A1<br>& JP 2005-509590 A | 1-3,7-9 |

Form PCT/ISA/210 (continuation of second sheet) (April 2005)

**INTERNATIONAL SEARCH REPORT**

| International application No. |
| --- |
| PCT/JP2005/016788 |

---

**Box No. II**     **Observations where certain claims were found unsearchable (Continuation of item 2 of first sheet)**

This international search report has not been established in respect of certain claims under Article 17(2)(a) for the following reasons:

1. ☒ Claims Nos.: 4-6
   because they relate to subject matter not required to be searched by this Authority, namely:
   ```
   Claims 4-6 pertain to a method for the treatment of a keratoconjunctiva
   disorder in which the carboxylic acid compound represented by any of the formulae
   (1) to (4) is used.  This subject matter falls under the category of methods
   (continued to extra sheet)
   ```

2. ☐ Claims Nos.:
   because they relate to parts of the international application that do not comply with the prescribed requirements to such an
   extent that no meaningful international search can be carried out, specifically:

3. ☐ Claims Nos.:
   because they are dependent claims and are not drafted in accordance with the second and third sentences of Rule 6.4(a).

---

**Box No. III**     **Observations where unity of invention is lacking (Continuation of item 3 of first sheet)**

This International Searching Authority found multiple inventions in this international application, as follows:
```
   Claims 1-3 and 7-9 of this application are considered to relate to the following
subject matters 1 to 3.
   1. A therapeutic agent for keratoconjunctiva disorders which contains as
an active ingredient at least one of oxyiminoalkanoic acid derivatives
represented by (1) and (2) shown in claim 1.
   2. A therapeutic agent for keratoconjunctiva disorders which contains a
carboxylic acid represented by (3) shown in claim 1.
   3. A therapeutic agent for keratoconjunctiva disorders which contains a
carboxylic acid represented by (4) shown in claim 1.
   (continued to extra sheet)
```

1. ☐ As all required additional search fees were timely paid by the applicant, this international search report covers all searchable
   claims.

2. ☐ As all searchable claims could be searched without effort justifying an additional fee, this Authority did not invite payment of
   any additional fee.

3. ☐ As only some of the required additional search fees were timely paid by the applicant, this international search report covers
   only those claims for which fees were paid, specifically claims Nos.:

4. ☒ No required additional search fees were timely paid by the applicant.   Consequently, this international search report is
   restricted to the invention first mentioned in the claims; it is covered by claims Nos.: 1-3,7-9

**Remark on Protest**     ☐ The additional search fees were accompanied by the applicant's protest and, where applicable, the
payment of a protest fee..

☐ The additional search fees were accompanied by the applicant's protest but the applicable protest
fee was not paid within the time limit specified in the invitation.

☐ No protest accompanied the payment of additional search fees.

Form PCT/ISA/210 (continuation of first sheet (2)) (April 2005)

| INTERNATIONAL SEARCH REPORT | International application No. |
| --- | --- |
| | PCT/JP2005/016788 |

Continuation of Box No.II-1 of continuation of first sheet(2)

for treatment of the human body by therapy. Namely the claims relate to a subject matter for which this International Searching Authority is not required, under the provisions of Article 17(2)(a)(i) of the PCT and Rule 39.1(iv) of the Regulations under the PCT, to search.

Continuation of Box No.III of continuation of first sheet(2)

A therapeutic agent for keratoconjunctiva disorders containing a carboxylic acid, which is a matter common to the subject matters 1 to 3, is known (see, for example, JP 2003-160485 A (Toa Medicine Co., Ltd.), [claim 1]; and JP 2002-205944 A (Santen Pharmaceutical Co., Ltd.), [claim 1]). Consequently, the therapeutic agent for keratoconjunctiva disorders containing a carboxylic acid does not exceed the scope of the prior art. This common matter, i.e., a therapeutic agent for keratoconjunctiva disorders containing a carboxylic acid, is hence not regarded as a special technical feature in the meaning of the second sentence of Rule 13.2 of the Regulations under the PCT.
Therefore, the subject matters 1 to 3 are not considered to be a group of inventions so linked as to form a single general inventive concept. They do not comply with the requirement of unity of invention.

In preparing an international search report, a search through prior art documents was made only for the subject matter 1, i.e., "a therapeutic agent for keratoconjunctiva disorders which contains as an active ingredient at least one of oxyiminoalkanoic acid derivatives represented by (1) and (2) shown in claim 1."

Form PCT/ISA/210 (extra sheet) (April 2005)

## REFERENCES CITED IN THE DESCRIPTION

**Patent documents cited in the description**

- JP 3074532 B
- JP 3417582 B
- WO 2002100813 A

**Non-patent literature cited in the description**

- *Japanese Review of Clinical Ophthalmology,* 1992, vol. 46, 738-743
- *Ophthalmic Surgery,* 1992, vol. 5, 719-727
- **FUJIHARA et al.** *Invest. Ophthalmol. Vis. Sci.,* 2001, vol. 42 (1), 96-100
- **MURAKAMI et al.** *Journal of the eye,* 2004, vol. 21 (1), 87-90